# EUROPEAN PATENT APPLICATION

(11) **EP 4 632 070 A1**
(43) Date of publication of application: **15.10.2025**
(21) Application number: 23900748.7
(22) Date of filing: 08.12.2023
(51) Int. Cl.: C12N 15/55, C12N 9/14, C12P 7/40, C12P 7/62

(54) **PROTEIN HAVING POLYETHYLENE TEREPHTHALATE DECOMPOSING ACTIVITY AND METHOD FOR DECOMPOSING POLYETHYLENE TEREPHTHALATE**

(30) Priority: 09.12.2022 JP 2022197409
(71) Applicant: KIRIN HOLDINGS KABUSHIKI KAISHA, Nakano-ku, Tokyo 164-0001 (JP)
(72) Inventor: MATSUZAKI Takashi, Tokyo 164-0001 (JP); YAMAZAKI Fuhito, Tokyo 164-0001 (JP)
(74) Representative: Vossius & Partner Patentanwälte Rechtsanwälte mbB
(86) International application number: PCT/JP2023/044010
(87) International publication number: WO 2024/122636

(57) **Abstract**

The present invention relates to a protein consisting of an amino acid sequence in which at least one modification selected from the group consisting of the following [1] to [7] is introduced in the amino acid sequence represented by SEQ ID NO: 1:
[1] a modification in which the amino acid residue at a position 103 is substituted with a lysine residue,
[2] a modification in which the amino acid residue at a position 76 is substituted with a cysteine residue,
[3] a modification in which the amino acid residue at a position 144 is substituted with a cysteine residue,
[4] a modification in which the amino acid residue at a position 134 is substituted with a glycine residue,
[5] a modification in which the amino acid residue at a position 222 is substituted with a methionine residue,
[6] a modification in which the amino acid residue at a position 73 is substituted with a glutamine residue, and
[7] a modification in which the amino acid residue at a position 203 is substituted with a threonine residue.

## Description

### TECHNICAL FIELD

The present invention relates to a protein having a polyethylene terephthalate decomposing activity, a DNA encoding the protein, a transformant obtained by transforming a host cell with the DNA, a method for decomposing polyethylene terephthalate, and a method for producing at least one of terephthalic acid and monohydroxyethyl terephthalate.

### BACKGROUND ART

Polyethylene terephthalate (hereinafter, also referred to as "PET") is widely used in beverage bottles, fibers, packaging materials, and the like. Since polyethylene terephthalate is not decomposed in the environment and it causes an environmental pollution, a PET recycling technique is required. Among the PET recycling technique, mechanical recycling has a problem in that the use of PET after decomposition is limited since impurities are not sufficiently removed. On the other hand, in chemical recycling, since PET can be decomposed and repolymerized in monomer units, impurities are easily removed, and the chemical recycling attracts attention as a sustainable recycling technique.

Among the chemical recycling, a PET decomposition method using an enzyme can be said to be an environmentally friendly recycling method since the reaction proceeds under mild conditions and an organic solvent is not used. As a PET decomposing enzyme, there have been found, for example, PETase derived from Ideonella sakaiensis (Patent Literature 1) and Cutinase derived from Thermobifida fusca (Patent Literature 2). LC-Cutinase derived from a leaf compost metagenome has a high decomposing activity and heat resistance (Patent Literature 3), and it has been reported that through enzyme modification, 97% or more of PET can be decomposed within 24 hours (Non Patent Literature 1). In a report of searching for a PET decomposing enzyme from metagenome, PET2 having relatively high thermal resistance has been found among enzymes, and it is also known that PET can be decomposed even in a small amount (Non Patent Literature 2). Further, there is a report that a decomposing activity and thermal resistance are improved by modification of PET2 (Non Patent Literature 3).

### CITATION LIST

### PATENT LITERATURE

Patent Literature 1: WO2015/025861
Patent Literature 2: Japanese Patent No. 6315978
Patent Literature 3: WO2012/099018

### NON-PATENT LITERATURE

Non Patent Literature 1: Nature, 2020, 580, 9, p.216-219
Non Patent Literature 2: Appl. Environ. Microbiol, 2018, 84, 8, p.e02773-17
Non Patent Literature 3: ACS Catal. 2021, 11, p.8550-8564

### SUMMARY OF INVENTION

### TECHNICAL PROBLEM

The PET decomposing enzymes described in Patent Literatures 1 and 2 have been researched and developed but have not yet been put into practical use, and the present inventors have studied and found that both of them have a low decomposing activity and low thermal resistance. As a result of studies by the present inventors, even when the enzymes described in Patent Literature 3 and Non Patent Literatures 1 to 3 are used, development of a PET decomposing enzyme having a higher activity in industrial applications is desired since it takes a long time to decompose PET by an enzyme reaction.

Accordingly, an object of the present invention is to provide a protein having a high PET decomposing activity.

### SOLUTION TO PROBLEM

As a result of intensive studies on the above problems, the present inventors have found that a protein consisting of an amino acid sequence in which at least one modification in which an amino acid residue at a specific position in SEQ ID NO: 1 is substituted with a specific amino acid residue is introduced in the amino acid sequence represented by SEQ ID NO: 1 has a PET decomposing activity higher than that of a protein comprising the amino acid sequence represented by SEQ ID NO: 1, and have completed the present invention.

That is, the present invention is as follows.
<1> A protein consisting of an amino acid sequence in which at least one modification selected from the group consisting of the following [1] to [7] is introduced in the amino acid sequence represented by SEQ ID NO: 1:
   [1] a modification in which the amino acid residue at a position 103 is substituted with a lysine residue,
   [2] a modification in which the amino acid residue at a position 76 is substituted with a cysteine residue,
   [3] a modification in which the amino acid residue at a position 144 is substituted with a cysteine residue,
   [4] a modification in which the amino acid residue at a position 134 is substituted with a glycine residue,
   [5] a modification in which the amino acid residue at a position 222 is substituted with a methionine residue,
   [6] a modification in which the amino acid residue at a position 73 is substituted with a glutamine residue, and
   [7] a modification in which the amino acid residue at a position 203 is substituted with a threonine residue.
<2> A protein consisting of an amino acid sequence in which at least one modification selected from the group consisting of the following [1'] to [7'] is introduced in an amino acid sequence of a mutant protein (B) having a modification which is at least one of deletion, substitution, insertion, and addition of 1 to 20 amino acid residues with respect to the amino acid sequence represented by SEQ ID NO: 1 and having a polyethylene terephthalate (PET) decomposing activity higher than that of the mutant protein (B):
   [1'] a modification in which an amino acid residue corresponding to the amino acid residue at a position 103 in SEQ ID NO: 1 is substituted with a lysine residue,
   [2'] a modification in which an amino acid residue corresponding to the amino acid residue at a position 76 in SEQ ID NO: 1 is substituted with a cysteine residue,
   [3'] a modification in which an amino acid residue corresponding to the amino acid residue at a position 144 in SEQ ID NO: 1 is substituted with a cysteine residue,
   [4'] a modification in which an amino acid residue corresponding to the amino acid residue at a position 134 in SEQ ID NO: 1 is substituted with a glycine residue,
   [5'] a modification in which an amino acid residue corresponding to the amino acid residue at a position 222 in SEQ ID NO: 1 is substituted with a methionine residue,
   [6'] a modification in which an amino acid residue corresponding to the amino acid residue at a position 73 in SEQ ID NO: 1 is substituted with a glutamine residue, and
   [7'] a modification in which an amino acid residue corresponding to the amino acid residue at a position 203 in SEQ ID NO: 1 is substituted with a threonine residue.
<3> A protein consisting of an amino acid sequence in which at least one modification selected from the group consisting of the following [1"] to [7"] is introduced in an amino acid sequence of a homologous protein (C) having 80% or more identity with the amino acid sequence represented by SEQ ID NO: 1 and having a polyethylene terephthalate (PET) decomposing activity higher than that of the homologous protein (C):
   [1"] a modification in which an amino acid residue corresponding to the amino acid residue at a position 103 in SEQ ID NO: 1 is substituted with a lysine residue,
   [2"] a modification in which an amino acid residue corresponding to the amino acid residue at a position 76 in SEQ ID NO: 1 is substituted with a cysteine residue,
   [3"] a modification in which an amino acid residue corresponding to the amino acid residue at a position 144 in SEQ ID NO: 1 is substituted with a cysteine residue,
   [4"] a modification in which an amino acid residue corresponding to the amino acid residue at a position 134 in SEQ ID NO: 1 is substituted with a glycine residue,
   [5"] a modification in which an amino acid residue corresponding to the amino acid residue at a position 222 in SEQ ID NO: 1 is substituted with a methionine residue,
   [6"] a modification in which an amino acid residue corresponding to the amino acid residue at a position 73 in SEQ ID NO: 1 is substituted with a glutamine residue, and
   [7"] a modification in which an amino acid residue corresponding to the amino acid residue at a position 203 in SEQ ID NO: 1 is substituted with a threonine residue.
<4> A DNA encoding the protein according to any one of <1> to <3>.
<5> A recombinant DNA comprising the DNA according to <4>.
<6> A transformant obtained by transforming a host cell with the recombinant DNA according to <5>.
<7> A method for decomposing PET using the protein according to any one of <1> to <3>.
<8> The method for decomposing PET according to <7>, including:
   decomposing the PET in a reaction solution containing the protein according to any one of <1> to <3>, the PET, magnesium chloride, and sodium carbonate.
<9> The method for decomposing PET according to <8>, in which
   a concentration of the magnesium chloride in the reaction solution is 0.1 mM to 5 mM.
<10> A method for producing at least one of terephthalic acid (TPA) and monohydroxyethyl terephthalate (MHET), the method including:
   decomposing PET using the protein according to any one of <1> to <3>.
<11> The method for producing at least one of TPA and MHET according to claim 10, including:
   decomposing the PET in a reaction solution containing the protein according to any one of <1> to <3>, the PET, magnesium chloride, and sodium carbonate.
<12> The method for producing at least one of TPA and MHET according to <11>, in which
   a concentration of the magnesium chloride in the reaction solution is 0.1 mM to 5 mM.

### ADVANTAGEOUS EFFECTS OF INVENTION

Since the protein according to one aspect of the present invention includes amino acid residue substitution at a specific site, the protein has a PET decomposing activity higher than that of a protein having no amino acid residue substitution.

### DESCRIPTION OF EMBODIMENTS

Hereinafter, the present invention will be described in detail, but these are examples of preferred embodiments and are not limited to these contents.

In the numerical range, "to" is a range including numerical values before and after that, and for example, "0 mass% to 100 mass%" means a range of 0 mass% or more and 100 mass% or less.

### 1. Protein having PET Decomposing Activity

A protein of one aspect of the present invention is consisting of an amino acid sequence in which at least one modification selected from the group consisting of the following [1] to [7] is introduced in the amino acid sequence represented by SEQ ID NO: 1:
[1] a modification in which the amino acid residue at a position 103 is substituted with a lysine residue
[2] a modification in which the amino acid residue at a position 76 is substituted with a cysteine residue
[3] a modification in which the amino acid residue at a position 144 is substituted with a cysteine residue
[4] a modification in which the amino acid residue at a position 134 is substituted with a glycine residue
[5] a modification in which the amino acid residue at a position 222 is substituted with a methionine residue
[6] a modification in which the amino acid residue at a position 73 is substituted with a glutamine residue
[7] a modification in which the amino acid residue at a position 203 is substituted with a threonine residue.

A protein consisting of the amino acid sequence represented by SEQ ID NO: 1 is the PET2 enzyme described in Non Patent Literature 1.

It is considered that when the protein is consisting of an amino acid sequence in which at least one modification selected from the group consisting of the above [1] to [7] is introduced in the amino acid sequence represented by SEQ ID NO: 1, at least one of heat resistance and a binding ability to a substrate is improved, and thus the protein shows a PET decomposing activity higher than that of a protein (A) comprising the amino acid sequence represented by SEQ ID NO: 1.

Examples of a non-limiting specific aspect of the protein of one aspect of the present invention include a protein consisting of an amino acid sequence in which any of the following (A1) to (A7) is introduced in the amino acid sequence represented by SEQ ID NO: 1:
(A1) the above [1]
(A2) the above [1] and [2]
(A3) the above [1] and [3]
(A4) the above [1], [2], and [3]
(A5) the above [1], [2], [3], and [4]
(A6) the above [1], [2], [3], [4], and [5]
(A7) the above [1], [2], [3], [4], [5], and [6]
(A8) the above [1], [2], [3], [4], [5], [6], and [7].

Examples of a non-limiting specific aspect of the protein of one aspect of the present invention include a protein consisting of an amino acid sequence in which at least the modification [1] is introduced, and further, at least one of the modifications [2] to [7] is freely introduced in the amino acid sequence represented by SEQ ID NO: 1.

Examples of another non-limiting specific aspect of the protein of one aspect of the present invention include a protein consisting of an amino acid sequence in which at least the modifications [1] and [2] are introduced, and further, at least one of the modifications [3] to [7] is freely introduced in the amino acid sequence represented by SEQ ID NO: 1.

Examples of another non-limiting specific aspect of the protein of one aspect of the present invention include a protein consisting of an amino acid sequence in which at least the modifications [1] and [3] are introduced, and further, at least one of the modifications [2], and [4] to [7] is freely introduced in the amino acid sequence represented by SEQ ID NO: 1.

Examples of another non-limiting specific aspect of the protein of one aspect of the present invention include a protein consisting of an amino acid sequence in which at least the modifications [1], [2], and [3] are introduced, and further, at least one of the modifications [4] to [7] is freely introduced in the amino acid sequence represented by SEQ ID NO: 1.

Examples of another non-limiting specific aspect of the protein of one aspect of the present invention include a protein consisting of an amino acid sequence in which at least the modifications [1], [2], [3], and [4] are introduced, and further, at least one of the modifications [5] to [7] is freely introduced in the amino acid sequence represented by SEQ ID NO: 1.

Examples of another non-limiting specific aspect of the protein of one aspect of the present invention include a protein consisting of an amino acid sequence in which at least the modifications [1], [2], [3], [4], and [5] are introduced, and further, at least one of the modifications [6] and [7] is freely introduced in the amino acid sequence represented by SEQ ID NO: 1.

Examples of another non-limiting specific aspect of the protein of one aspect of the present invention include a protein consisting of an amino acid sequence in which at least the modifications [1], [2], [3], [4], [5], and [6] are introduced, and further, the modification [7] is freely introduced in the amino acid sequence represented by SEQ ID NO: 1.

Examples of another non-limiting specific aspect of the protein of one aspect of the present invention include a protein consisting of an amino acid sequence in which the modifications [1], [2], [3], [4], [5], [6], and [7] are introduced in the amino acid sequence represented by SEQ ID NO: 1.

A protein of one aspect of the present invention is consisting of an amino acid sequence in which at least one modification selected from the group consisting of the following [1'] to [7'] is introduced in an amino acid sequence of a mutant protein (B) having a modification including at least one of deletion, substitution, insertion, and addition of 1 to 20 amino acid residues with respect to the amino acid sequence represented by SEQ ID NO: 1 and has a PET decomposing activity higher than that of the mutant protein (B):
[1'] a modification in which an amino acid residue corresponding to the amino acid residue at a position 103 in SEQ ID NO: 1 is substituted with a lysine residue
[2'] a modification in which an amino acid residue corresponding to the amino acid residue at a position 76 in SEQ ID NO: 1 is substituted with a cysteine residue
[3'] a modification in which an amino acid residue corresponding to the amino acid residue at a position 144 in SEQ ID NO: 1 is substituted with a cysteine residue
[4'] a modification in which an amino acid residue corresponding to the amino acid residue at a position 134 in SEQ ID NO: 1 is substituted with a glycine residue
[5'] a modification in which an amino acid residue corresponding to the amino acid residue at a position 222 in SEQ ID NO: 1 is substituted with a methionine residue
[6'] a modification in which an amino acid residue corresponding to the amino acid residue at a position 73 in SEQ ID NO: 1 is substituted with a glutamine residue
[7'] a modification in which an amino acid residue corresponding to the amino acid residue at a position 203 in SEQ ID NO: 1 is substituted with a threonine residue.

In the present description, the mutant protein refers to a protein obtained by artificially deleting or substituting an amino acid residue of an original protein or inserting or adding an amino acid residue in the protein.

The expression "the mutant protein has a modification including at least one of deletion, substitution, insertion, and addition of an amino acid" means that the mutant protein may have a modification including at least one of deletion, substitution, insertion and addition of 1 to 20 amino acids at any position in the amino acid sequence represented by SEQ ID NO: 1. The number of amino acids to be modified by at least one of deletion, substitution, insertion, and addition is 1 to 20, preferably 1 to 10, more preferably 1 to 8, and most preferably 1 to 5.

The amino acid to be modified by at least one of deletion, substitution, insertion, and addition may be natural or non-natural. Examples of the natural type amino acid include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

Examples of mutually substitutable amino acids are shown below. Amino acids contained in the same group can be mutually substituted.
Group A: leucine, isoleucine, norleucine, valine, norvaline, alanine, 2-aminobutanoic acid, methionine, O-methylserine, t-butylglycine, t-butylalanine, and cyclohexylalanine
Group B: aspartic acid, glutamic acid, isoaspartic acid, isoglutamic acid, 2-aminoadipic acid, and 2-aminosuberic acid
Group C: asparagine and glutamine
Group D: lysine, arginine, ornithine, 2,4-diaminobutanoic acid, and 2,3-diaminopropionic acid
Group E: proline, 3-hydroxyproline, and 4-hydroxyproline
Group F: serine, threonine, and homoserine
Group G: phenylalanine and tyrosine

Examples of a non-limiting specific aspect of the protein of one aspect of the present invention include a protein consisting of an amino acid sequence in which any of the following (B1) to (B8) is introduced in the amino acid sequence of the mutant protein (B) having a modification including at least one of deletion, substitution, insertion, and addition of 1 to 20 amino acid residues in the amino acid sequence represented by SEQ ID NO: 1:
(B1) the above [1']
(B2) the above [1'] and [2']
(B3) the above [1'] and [3']
(B4) the above [1'], [2'], and [3']
(B5) the above [1'], [2'], [3'], and [4']
(B6) the above [1'], [2'], [3'], [4'], and [5']
(B7) the above [1'], [2'], [3'], [4'], [5'], and [6']
(B8) the above [1'], [2'], [3'], [4'], [5'], [6'], and [7'].

Examples of a non-limiting specific aspect of the protein of one aspect of the present invention include a protein consisting of an amino acid sequence in which at least the modification [1'] is introduced, and further, at least one of the modifications [2'] to [7'] is freely introduced in the amino acid sequence of the mutant protein (B).

Examples of another non-limiting specific aspect of the protein of one aspect of the present invention include a protein consisting of an amino acid sequence in which at least the modifications [1'] and [2'] are introduced, and further, at least one of the modifications [3'] to [7'] is freely introduced in the amino acid sequence of the mutant protein (B).

Examples of another non-limiting specific aspect of the protein of one aspect of the present invention include a protein consisting of an amino acid sequence in which at least the modifications [1'] and [3'] are introduced, and further, at least one of the modifications [2'], and [4'] to [7'] is freely introduced in the amino acid sequence of the mutant protein (B).

Examples of another non-limiting specific aspect of the protein of one aspect of the present invention include a protein consisting of an amino acid sequence in which at least the modifications [1'], [2'], and [3'] are introduced, and further, at least one of the modifications [4'] to [7'] is freely introduced in the amino acid sequence of the mutant protein (B).

Examples of another non-limiting specific aspect of the protein of one aspect of the present invention include a protein consisting of an amino acid sequence in which at least the modifications [1'], [2'], [3'], and [4'] are introduced, and further, at least one of the modifications [5'] to [7'] is freely introduced in the amino acid sequence of the mutant protein (B).

Examples of another non-limiting specific aspect of the protein of one aspect of the present invention include a protein consisting of an amino acid sequence in which at least the modifications [1'], [2'], [3'], [4'], and [5'] are introduced, and further, at least one of the modifications [6'] and [7'] is freely introduced in the amino acid sequence of the mutant protein (B).

Examples of another non-limiting specific aspect of the protein of one aspect of the present invention include a protein consisting of an amino acid sequence in which at least the modifications [1'], [2'], [3'], [4'], [5'], and [6'] are introduced, and further, the modification [7'] is freely introduced in the amino acid sequence of the mutant protein (B).

Examples of another non-limiting specific aspect of the protein of one aspect of the present invention include a protein consisting of an amino acid sequence in which the modifications [1'], [2'], [3'], [4'], [5'], [6'], and [7'] are introduced in the amino acid sequence of the mutant protein (B).

A protein of one aspect of the present invention is consisting of an amino acid sequence in which at least one modification selected from the group consisting of the following [1"] to [7"] is introduced in an amino acid sequence of a homologous protein (C) having 80% or more identity with the amino acid sequence represented by SEQ ID NO: 1 and has a polyethylene terephthalate (PET) decomposing activity higher than that of the homologous protein (C):
[1"] a modification in which an amino acid residue corresponding to the amino acid residue at a position 103 in SEQ ID NO: 1 is substituted with a lysine residue
[2"] a modification in which an amino acid residue corresponding to the amino acid residue at a position 76 in SEQ ID NO: 1 is substituted with a cysteine residue
[3"] a modification in which an amino acid residue corresponding to the amino acid residue at a position 144 in SEQ ID NO: 1 is substituted with a cysteine residue
[4"] a modification in which an amino acid residue corresponding to the amino acid residue at a position 134 in SEQ ID NO: 1 is substituted with a glycine residue
[5"] a modification in which an amino acid residue corresponding to the amino acid residue at a position 222 in SEQ ID NO: 1 is substituted with a methionine residue
[6"] a modification in which an amino acid residue corresponding to the amino acid residue at a position 73 in SEQ ID NO: 1 is substituted with a glutamine residue
[7"] a modification in which an amino acid residue corresponding to the amino acid residue at a position 203 in SEQ ID NO: 1 is substituted with a threonine residue.

As used herein, the term "homologous protein" refers to a protein similar in structure and function to the original protein.

Examples of the homologous protein include an amino acid sequence having an identity of 80% or more, preferably 90% or more, and particularly preferably 95% or more with the amino acid sequence of a target protein.

The identity of an amino acid sequence and a nucleotide sequence can be determined by using an algorithm BLAST [Pro. Natl. Acad. Sci. USA, 90, 5873 (1993)] or FASTA [Methods Enzymol., 183, 63 (1990)] developed by Karlin and Altschul. Programs called BLASTN and BLASTX have been developed based on the algorithm BLAST [J. Mol. Biol., 215, 403 (1990)]. When the nucleotide sequence is analyzed by BLASTN based on BLAST, the parameters are, for example, Score = 100 and wordlength = 12. When the amino acid sequence is analyzed by BLASTX based on BLAST, the parameters are, for example, score = 50 and wordlength = 3. When BLAST program and Gapped BLAST programs are used, default parameters for each program are used. A specific method of the analysis methods is well known.

An alignment between the amino acid sequence represented by SEQ ID NO: 1 and the amino acid sequence of the homologous protein can be prepared using a known alignment program ClustalW [Nucelic Acids Research 22, 4673, (1994)]. ClustalW can be used by http://www.ebi.ac.uk/clustalw/(European Bioinformatics Institute). For example, a default value is used for parameters when creating an alignment using ClustalW.

Examples of a non-limiting specific aspect of the protein of one aspect of the present invention include a protein consisting of an amino acid sequence in which any of the following (C1) to (C8) is introduced in the amino acid sequence of the homologous protein (C) having 80% or more identity to the amino acid sequence represented by SEQ ID NO: 1:
(C1) the above [1"]
(C2) the above [1"] and [2"]
(C3) the above [1"] and [3"]
(C4) the above [1"], [2"], and [3"]
(C5) the above [1"], [2"], [3"], and [4"]
(C6) the above [1"], [2"], [3"], [4"], and [5"]
(C7) the above [1"], [2"], [3"], [4"], [5"], and [6"]
(C8) the above [1"], [2"], [3"], [4"], [5"], [6"], and [7"].

Examples of a non-limiting specific aspect of the protein of one aspect of the present invention include a protein consisting of an amino acid sequence in which at least the modification [1"] is introduced, and further, at least one of the modifications [2"] to [7"] is freely introduced in the amino acid sequence of the homologous protein (C).

Examples of another non-limiting specific aspect of the protein of one aspect of the present invention include a protein consisting of an amino acid sequence in which at least the modifications [1"] and [2"] are introduced, and further, at least one of the modifications [3"] to [7"] is freely introduced in the amino acid sequence of the homologous protein (C).

Examples of another non-limiting specific aspect of the protein of one aspect of the present invention include a protein consisting of an amino acid sequence in which at least the modifications [1"] and [3"] are introduced, and further, at least one of the modifications [2"] and [4"] to [7"] is freely introduced in the amino acid sequence of the homologous protein (C).

Examples of another non-limiting specific aspect of the protein of one aspect of the present invention include a protein consisting of an amino acid sequence in which at least the modifications [1"], [2"], and [3"] are introduced, and further, at least one of the modifications [4"] to [7"] is freely introduced in the amino acid sequence of the homologous protein (C).

Examples of another non-limiting specific aspect of the protein of one aspect of the present invention include a protein consisting of an amino acid sequence in which at least the modifications [1"], [2"], [3"], and [4"] are introduced, and further, at least one of the modifications [5"] to [7"] is freely introduced in the amino acid sequence of the homologous protein (C).

Examples of another non-limiting specific aspect of the protein of one aspect of the present invention include a protein consisting of an amino acid sequence in which at least the modifications [1"], [2"], [3"], [4"], and [5"] are introduced, and further, at least one of the modifications [6"] and [7"] is freely introduced in the amino acid sequence of the homologous protein (C).

Examples of another non-limiting specific aspect of the protein of one aspect of the present invention include a protein consisting of an amino acid sequence in which at least the modifications [1"], [2"], [3"], [4"], [5"], and [6"] are introduced, and further, the modification [7"] is freely introduced in the amino acid sequence of the homologous protein (C).

Examples of another non-limiting specific aspect of the protein of one aspect of the present invention include a protein consisting of an amino acid sequence in which the modifications [1"], [2"], [3"], [4"], [5"], [6"], and [7"] are introduced in the amino acid sequence of the homologous protein (C).

The protein of one aspect of the present invention can decompose a main chain of PET. Therefore, the protein of one aspect of the present invention can decompose bis(2-hydroxyethyl)terephthalate (hereinafter, also referred to as "BHET"), which is an intermediate product of PET decomposition, into monohydroxyethyl terephthalate (hereinafter, also referred to as "MHET"), and further decompose the MHET into terephthalic acid (hereinafter, also referred to as "TPA") and ethylene glycol (hereinafter, also referred to as "EG"). That is, the protein of one aspect of the present invention can hydrolyze PET or BHET, which is a partial structure of PET, as a substrate to produce MHET, and further produce TPA and EG.

The PET decomposing activity of a protein can be confirmed, for example, by preparing a reaction solution containing the protein and a PET film fragment, performing an enzyme reaction, and measuring amounts of TPA and MHET generated after the completion of the reaction. When a total amount of TPA and MHET produced from the reaction solution containing the protein of one aspect of the present invention and the PET film fragment is larger than a total amount of TPA and MHET produced from the reaction solution containing a protein of a comparison target and a PET film fragment, it can be said that the protein of one aspect of the present invention has a PET decomposing activity higher than that of the protein of the comparison target.

The amounts of generated TPA and MHET can be measured by a high-performance liquid chromatograph (hereinafter, also referred to as "HPLC").

The protein of one aspect of the present invention is also referred to as a mutant-type PET2 according to the present embodiment.

### 2. DNA

Examples of a DNA of one aspect of the present invention include a DNA encoding the protein of one aspect of the present invention described in 1. above.

### 3. Transformant

Examples of a transformant transformed with the DNA encoding the protein of one aspect of the present invention include a transformant obtained by transforming a host cell by a known method using a recombinant DNA containing the DNA in 2. above. The host cell may be any of a prokaryote, yeast, an animal cell, an insect cell, a plant cell, and the like, and is preferably a prokaryote such as a bacterium, and more preferably a microorganism belonging to the genera Escherichia, Serratia, Bacillus, Brevibacterium, Corynebacterium, Microbacterium, Pseudomonas, and the like.

### 4. Preparation of DNA of One Aspect of Present Invention

A DNA (a) encoding the protein consisting of an amino acid sequence in which at least one modification selected from the group consisting of the above [1] to [7] is introduced in the amino acid sequence represented by SEQ ID NO: 1, which is the DNA of one aspect of the present invention, can be obtained by the following method.

The above DNA (a) can be obtained by using a DNA encoding the protein consisting of the amino acid sequence represented by SEQ ID NO: 1 and substituting a nucleotide sequence of a portion encoding an amino acid residue before substitution with a nucleotide sequence encoding an amino acid residue after substitution in at least one modification site selected from the group consisting of the above [1] to [7] described in 1. above, for example, by a site-directed mutagenesis method described in Molecular Cloning, 3rd Edition, and Current Protocols in Molecular Biology, and the like.

A DNA (b) encoding the protein consisting of an amino acid sequence in which at least one modification selected from the above [1'] to [7'] is introduced in the amino acid sequence of the mutant protein (B) having a modification including at least one of deletion, substitution, insertion, and addition of 1 to 20 amino acid residues with respect to the amino acid sequence represented by SEQ ID NO: 1 and having a PET decomposing activity higher than that of the mutant protein (B), which is the DNA of one aspect of the present invention, can be obtained by the following method.

The DNA (b) can be obtained by using a DNA encoding the mutant protein (B) having a modification including at least one of deletion, substitution, insertion, and addition of 1 to 20 amino acid residues in the amino acid sequence represented by SEQ ID NO: 1, aligning the amino acid sequence represented by SEQ ID NO: 1 and the mutant protein (B) by the method described in 1. above, and substituting a nucleotide sequence of a portion encoding an amino acid residue before substitution with a nucleotide sequence encoding an amino acid residue after substitution in at least one modification site selected from the group consisting of the above [1'] to [7'] described in 1. above in the amino acid sequence of the mutant protein (B) by the site-directed mutagenesis method.

The DNA (b) can also be obtained by a method using a DNA encoding the protein consisting of an amino acid sequence in which at least one modification selected from the group consisting of the above [1] to [7] is introduced in the amino acid sequence represented by SEQ ID NO: 1, and introducing a mutation into a nucleotide sequence of a portion encoding 1 to 20 amino acid residues such that a modification including at least one of deletion, substitution, insertion, and addition of the 1 to 20 amino acid residues at a site other than a site of the modification is made.

The DNA (b) can also be obtained by a method using a DNA encoding the protein consisting of the amino acid sequence represented by SEQ ID NO: 1, and introducing a mutation into a nucleotide sequence of a portion encoding modification sites such that a modification selected from the group consisting of the above [1'] to [7'] and a modification including at least one of deletion, substitution, insertion, and addition of 1 to 20 amino acid residues at a site other than a site of the modification are made.

In the above acquisition method, a primer designed to allow introduction of a target modification can be used.

A DNA (c) encoding the protein consisting of an amino acid sequence in which at least one modification selected from the group consisting of the above [1"] to [7"] is introduced in the amino acid sequence of the homologous protein (C) having 80% or more identity with the amino acid sequence represented by SEQ ID NO: 1 and having a PET decomposing activity higher than that of the homologous protein (C) can be obtained by the following method.

The DNA (c) can be obtained by using a DNA encoding the homologous protein (C) having 80% or more identity with the amino acid sequence represented by SEQ ID NO: 1, aligning the amino acid sequence represented by SEQ ID NO: 1 and the homologous protein (C) by the method described in 1. above, and substituting a nucleotide sequence of a portion encoding an amino acid residue before substitution with a nucleotide sequence encoding an amino acid residue after substitution in at least one modification site selected from the group consisting of the above [1"] to [7"] described in 1. above in the amino acid sequence of the homologous protein (C) by the site-directed mutagenesis method.

The DNA (c) can also be obtained by using a DNA encoding the protein consisting of an amino acid sequence in which at least one modification selected from the group consisting of the above [1] to [7] is introduced in the amino acid sequence represented by SEQ ID NO: 1, and introducing a modification into a nucleotide sequence of a portion encoding a site other than the modification site. At this time, the modification site is designed such that the amino acid sequence other than at least one modification site selected from the group consisting of the above [1] to [7] has 80% or more identity with the amino acid sequence represented by SEQ ID NO: 1.

The DNA of one aspect of the present invention can be obtained by using the DNA encoding the protein consisting of the amino acid sequence represented by SEQ ID NO: 1, and introducing at least one modification selected from the group consisting of the above [1"] to [7"] and a modification into a nucleotide sequence of a portion encoding a site other than the modification site. At this time, the modification site is designed such that the amino acid sequence other than at least one modification site selected from the group consisting of the above [1"] to [7"] has 80% or more identity with the amino acid sequence represented by SEQ ID NO: 1.

In the above acquisition method, a primer designed to allow introduction of a target modification can be used.

### 5. Production of Transformant of One Aspect of Present Invention

Examples of a transformant of one aspect of the present invention include a transformant obtained by introducing a recombinant DNA comprising the DNA of one aspect of the present invention into a host cell, and a transformant obtained by introducing a recombinant DNA obtained by incorporating the DNA of one aspect of the present invention into a vector DNA into a host cell.

Examples of the vector into which the DNA of the present invention is incorporated include pET28a (manufactured by Sigma-Aldrich), pBluescriptII KS(+) (manufactured by Stratagene), pDIRECT [Nucleic Acids Res., 18, 6069 (1990)], pCR-Script Amp SK(+) (manufactured by Stratagene), pT7Blue (manufactured by Novagen), pCR II (manufactured by Invitrogen), pCR-TRAP (manufactured by Gene Hunter), and pQE-60 (manufactured by Qiagen).

Examples of the host cell include a microorganism belonging to the genus Escherichia. Examples of the microorganism belonging to the genus Escherichia include Escherichia coli SHuffle T7 Express Competent, Escherichia coli XL1-Blue, Escherichia coli XL2-Blue, Escherichia coli DH1, Escherichia coli DH5α, Escherichia coli MC1000, Escherichia coli ATCC 12435, Escherichia coli W1485, Escherichia coli JM109, Escherichia coli HB101, Escherichia coli No. 49, Escherichia coli W3110, Escherichia coli NY49, Escherichia coli MP347, Escherichia coli NM522, Escherichia coli BL21, Escherichia coli ME8415, and the like.

As a method for introducing the recombinant DNA, any method for introducing a DNA into a host cell can be used, and examples thereof include a method using calcium ions [Proc. Natl. Acad. Sci., USA, 69, 2110 (1972)], a protoplast method (JPS63-248394A), and an electroporation method [Nucleic Acids Res., 16, 6127 (1988)].

### 6. Method for Producing Protein of One Aspect of Present Invention

### (1) Production of Transformant Producing Protein of One Aspect of Present Invention

Based on the DNA of one aspect of the present invention, a DNA fragment of an appropriate length comprising a portion encoding the protein of one aspect of the present invention is prepared as necessary. By substituting bases in the nucleotide sequence of the portion encoding the protein such that the nucleotide sequence contains codons optimal for expression in the host, a transformant with improved production efficiency of the protein can be obtained.

By introducing the recombinant DNA into a host cell compatible with the expression vector, a transformant producing the protein of one aspect of the present invention can be obtained.

As the host cell, any cell capable of expressing a target gene can be used, including a bacterium, yeast, an animal cell, an insect cell, a plant cell, and the like.

As the expression vector, an expression vector that can autonomously replicate in the host cell or can be incorporated into the chromosome and contains a promoter at a position where the DNA of the present invention can be transcribed is used.

When a prokaryote such as a bacterium is used as the host cell, the recombinant DNA comprising the DNA of the present invention can autonomously replicate in the prokaryote, and, at the same time, is preferably a recombinant DNA composed of a promoter, a ribosome binding sequence, the DNA of the present invention, and a transcription termination sequence, and may contain a gene for controlling the promoter.

Examples of the expression vector include pET28a (manufactured by Sigma-Aldrich), pColdI (manufactured by Takara Bio Inc.), pCDF-1b and pRSF-1b (both manufactured by Novagen), pMAL-c2x (manufactured by New England Biolabs), pGEX-4T-1 (manufactured by GE Healthcare Biosciences), pTrcHis (manufactured by Invitrogen), pSE280 (manufactured by Invitrogen), pGEMEX-1 (manufactured by Promega), pQE-30 (manufactured by Qiagen), pQE-60 (manufactured by Qiagen), pET-3 (manufactured by Novagen), pKYP10 (JPS58-110600A), pKYP200 [Agric. Biol. Chem., 48, 669 (1984)], pLSA1 [Agric. Biol. Chem., 53, 277 (1989)], pGEL1 [Proc. Natl. Acad. Sci., USA, 82, 4306 (1985)], pBluescript II SK(+) and pBluescript II KS(-) (manufactured by Stratagene), pTrS30 [prepared from Escherichia coli JM109/pTrS30 (FERM BP-5407)], pTrS32 [prepared from Escherichia coli JM109/pTrS32 (FERM BP-5408)], pPAC31 (WO98/12343), pUC19 [Gene, 33, 103 (1985)], pSTV28 (manufactured by Takara Bio Inc.), pUC118 (manufactured by Takara Bio Inc.), pPA1 (JPS63-233798A), pGHA2 [prepared from Escherichia coli IGHAA2 (FERM B-400), JPS60-221091A], pGKA2 [prepared from Escherichia coli IGKA2 (FERM BP-6798), JPS60-221091A], pTerm2 (US4686191B, US4939094B; US5160735B), pSupex, pUB110, pTP5, pC194, and pEG400 [J. Bacteriol., 172, 2392 (1990)], pCG1 (JPS57-134500A), pCG2 (JPS58-35197A), pCG4 (JPS57-183799A), pCG11 (JPS57-134500A), pCG116, pCE54, and pCB101 (all from JPS58-105999A), pRI109 (WO00/044886), and pCE51, pCE52, pCE53 [all from Molecular and General Genetics, 196, 175 (1984)], and the like.

The promoter may be any promoter that functions in the host cell such as Escherichia coli. Examples thereof include a promoter derived from Escherichia coli or a phage, such as the trp promoter (Ptrp), the lac promoter (Plac), the PL promoter, the PR promoter, and the PSE promoter, the SPO1 promoter, the SPO2 promoter, the penP promoter, and the like. Artificially designed and modified promoters such as a promoter with two Ptrp promoters in series (Ptrp ×2), the tac promoter, the lacT7 promoter, and the let I promoter can also be used.

It is preferable to use a plasmid in which a distance between a Shine-Dalgarno sequence, which is a ribosome binding sequence, and a start codon is adjusted to an appropriate distance (for example, 6 to 18 bases). Although a transcription termination sequence is not necessarily required for expression of the DNA of one aspect of the present invention, it is preferable to place a transcription termination sequence immediately downstream of the structural gene in the recombinant DNA.

Examples of the prokaryote include a microorganism belonging to the genera Escherichia, Serratia, Bacillus, Brevibacterium, Corynebacterium, Microbacterium, Pseudomonas, and the like, such as Escherichia coli SHuffle T7 Express Competent, Escherichia coli XL1-Blue, Escherichia coli XL2-Blue, Escherichia coli DH1, Escherichia coli DH5α, Escherichia coli NM522, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Serratia ficaria, Serratia fonticola, Serratia liquefaciens, Serratia marcescens, Bacillus subtilis, Bacillus amyloliquefaciens, Brevibacterium immariophilum ATCC14068, Brevibacterium saccharolyticum ATCC14066, Corynebacterium ammoniagenes, Corynebacterium glutamicum ATCC13032, Corynebacterium glutamicum ATCC14067, Corynebacterium glutamicum ATCC13869, Corynebacterium acetoacidophilum ATCC13870, Microbacterium ammoniaphilum ATCC15354, and Pseudomonas sp. D-0110. Preferred examples thereof include a microorganism belonging to the genus Escherichia or Corynebacterium, and more preferred examples thereof include Escherichia coli XL1-Blue, Escherichia coli XL2-Blue, Escherichia coli DH1, Escherichia coli DH5α, Escherichia coli MC1000, Escherichia coli MM294, Escherichia coli W1485, Escherichia coli JM109, Escherichia coli HB101, Escherichia coli No. 49, Escherichia coli W3110, Escherichia coli NY49, Escherichia coli GI698, Corynebacterium ammoniagenes ATCC6872, Corynebacterium ammoniagenes ATCC21170, Corynebacterium glutamicum ATCC13032, Corynebacterium glutamicum ATCC21171, and the like.

The method for introducing a recombinant vector can be any method for introducing a DNA into a host cell, and examples thereof include a method using calcium ions [Proc. Natl. Acad. Sci. USA, 69, 2110 (1972)], a protoplast method (JPS57-186492A and JPS57-18649A), electroporation [for example, Journal of Bacteriology, 175, 4096 (1993); Appl. Microbiol. Biotechnol., 52, 541 (1999)], a method described in Gene, 17, 107 (1982) and Molecular & General Genetics, 168, 111 (1979), and the like.

When a yeast strain is used as the host cell, examples of the expression vector include YEp13 (ATCC 37115), YEp24 (ATCC 37051), YCp50 (ATCC 37419), pHS19, pHS15, and the like.

As the promoter, any promoter may be used as long as it functions in a yeast strain, and examples thereof include the PHO5 promoter, the PGK promoter, the GAP promoter, the ADH promoter, the gal 1 promoter, the gal 10 promoter, the heat shock polypeptide promoter, the MFα1 promoter, the CUP 1 promoter, and the like.

Examples of the host cell include a yeast strain belonging to the genera Saccharomyces, Schizosaccharomyces, Kluyveromyces, Trichosporon, Siwanniomyces, Pichia, Candida, and the like. Specific examples thereof include Saccharomyces cerevisiae, Schizosaccharomyces pombe, Kluyveromyces lactis, Trichosporon pullulans, Schwanniomyces alluvius, Pichia pastoris, Candida utilis, and the like.

As a method for introducing the recombinant DNA, any method for introducing a DNA into yeast can be used, and examples thereof include an electroporation method (Methods Enzymol., 194, 182 (1990)), a spheroplast method (Proc. Natl. Acad. Sci., USA, 81, 4889 (1984)), a lithium acetate method (J. Bacteriol., 153, 163 (1983)), and the like.

### (2) Method for Producing Protein of One Aspect of Present Invention

The protein can be produced by culturing the transformant obtained by the method (1) in a medium, producing and accumulating the protein of one aspect of the present invention in a culture, and collecting the protein from the culture.

The host for the above-described transformant for producing the protein of one aspect of the present invention may be any of a prokaryote, yeast, an animal cell, an insect cell, a plant cell, and the like, and is preferably a prokaryote such as a bacterium, and more preferably a microorganism belonging to the genera Escherichia, Serratia, Bacillus, Brevibacterium, Corynebacterium, Microbacterium, Pseudomonas, and the like.

When expressed in yeast, an animal cell, an insect cell, or a plant cell, a protein having sugar or a sugar chain added thereto can be obtained.

The method for culturing the transformant in a medium can be performed according to a method generally used for culturing a host.

As a medium for culturing a transformant obtained using a prokaryote such as Escherichia coli or a eukaryote such as yeast as a host, any of a natural medium and a synthetic medium may be used as long as the medium contains a carbon source, a nitrogen source, an inorganic salt, and the like that can be assimilated by the transformant and can efficiently culture the transformant.

The carbon source is not particularly limited as long as it can be assimilated by the organism, and examples thereof include carbohydrates such as glucose, fructose, sucrose, molasses containing the same, starch, and starch hydrolysates; organic acids such as acetic acid and propionic acid; alcohols such as ethanol and propanol; and the like.

Examples of the nitrogen source include ammonia, ammonium salts of inorganic acids or organic acids, such as ammonium chloride, ammonium sulfate, ammonium acetate, and ammonium phosphate, and other nitrogen-containing compounds, as well as peptones, meat extracts, yeast extracts, corn steep liquor, casein hydrolysates, soybean meal and soybean meal hydrolysates, various fermented bacterial cells, digested products thereof, and the like.

Examples of the inorganic salt include potassium primary phosphate, potassium secondary phosphate, magnesium phosphate, magnesium sulfate, sodium chloride, ferrous sulfate, manganese sulfate, copper sulfate, calcium carbonate, and the like.

Culture is generally performed under aerobic conditions such as shaking culture or deep aeration stirring culture. A culture temperature is 15°C to 40°C, and a culture time is generally 5 hours to 7 days. A pH during the culture is maintained at 3.0 to 11. The pH is adjusted using an inorganic or organic acid, an alkaline solution, urea, calcium carbonate, ammonia, or the like.

During the culture, antibiotics such as kanamycin, ampicillin, and tetracycline may be added to the medium, if necessary.

When a microorganism transformed with an expression vector is cultured using an inducible promoter as a promoter, an inducer may be added to the culture medium as necessary. For example, isopropyl-β-D-thiogalactopyranoside or the like may be added to the culture medium when a microorganism transformed with an expression vector using a lac promoter is cultured, and indoleacrylic acid or the like may be added to the culture medium when a microorganism transformed with an expression vector using a trp promoter is cultured.

Examples of the method for producing the protein of one aspect of the present invention include a method for producing a protein in a host cell, a method for secreting a protein out of a host cell, and a method for producing a protein on an outer membrane of a host cell, and a structure of a protein to be produced can be changed depending on the selected method.

When the protein of one aspect of the present invention is produced in a host cell or on an outer membrane of a host cell, the protein can be actively secreted outside the host cell by applying a method described by Paulson et al. [J. Biol. Chem., 264, 17619 (1989)], a method described by Rowe et al. [Proc. Natl. Acad. Sci., USA, 86, 8227 (1989); Genes Develop., 4, 1288 (1990)], or methods described in JPH05-336963A and WO94/23021.

That is, by producing the protein of one aspect of the present invention in a form in which a signal peptide is added before a protein containing an active site thereof using a genetic recombination technique, the protein can be actively secreted outside the host cell.

A production amount can also be increased by using a gene amplification system using a dihydrofolate reductase gene or the like according to a method described in JPH2-227075A.

In order to isolate and purify the protein produced by the transformant of the present invention, a general enzyme isolation and purification method can be used.

For example, when the protein of one aspect of the present invention is expressed in a lytic state within cells, the cells are collected by centrifugation after completion of culture, suspended in an aqueous buffer solution, and then disrupted by an ultrasonic crusher, a French press, a Manton Gaulin homogenizer, a Dyno-Mill, or the like to obtain a cell-free extract. From a supernatant obtained by centrifuging the cell-free extract, a purified preparation can be obtained by using a general enzyme isolation and purification method, that is, a solvent extraction method, a salting-out method using ammonium sulfate or the like, a desalting method, a precipitation method using an organic solvent, an anion exchange chromatography method using a resin such as diethylaminoethyl (DEAE)-Sepharose or DIAION (registered trademark) HPA-75 (manufactured by Mitsubishi Chemical Corporation), a cation exchange chromatography method using a resin such as S-Sepharose FF (manufactured by Amersham Biosciences), a hydrophobic chromatography method using a resin such as butyl sepharose or phenyl sepharose, a gel filtration method using a molecular sieve, an affinity chromatography method, a chromatofocusing method, an electrophoresis method such as isoelectric focusing, or the like alone or in combination.

When the protein is expressed by forming an insoluble body within cells, the cells are similarly collected, disrupted, and centrifuged to collect the insoluble body of the protein as a precipitate fraction. The collected insoluble body is solubilized with a protein denaturing agent. The solubilized solution is diluted or dialyzed to reduce a concentration of the protein denaturing agent in the solubilized solution, thereby restoring the protein to a normal conformation. After this operation, a purified preparation of the protein can be obtained by the same isolation and purification method as described above.

When the protein of one aspect of the present invention or a derivative such as a protein obtained by adding a sugar chain to the protein is secreted extracellularly, the protein or the derivative of the protein can be collected from a culture supernatant. That is, the culture supernatant is obtained by treating the culture by the same method such as centrifugation as described above, and a purified preparation can be obtained from the culture supernatant by using the same isolation and purification method as described above.

Examples of the protein thus obtained include the protein of one aspect of the present invention.

The protein of one aspect of the present invention can also be produced as a fusion protein with another protein and purified by affinity chromatography using a substance having affinity for the fused protein. For example, the protein of one aspect of the present invention can be produced as a fusion protein with protein A and purified by affinity chromatography using immunoglobulin G, in accordance with a method described by Lowe et al. [Proc. Natl. Acad. Sci., USA, 86, 8227 (1989), Genes Develop., 4, 1288 (1990)], JPH5-336963A, and WO94/23021.

The protein of one aspect of the present invention can also be produced as a fusion protein with a Flag peptide and purified by affinity chromatography using an anti-Flag antibody [Proc. Natl. Acad. Sci., USA, 86, 8227 (1989), Genes Develop., 4, 1288 (1990)] or by affinity chromatography using a metal coordination resin having a high affinity for polyhistidine. Further, the protein can also be purified by affinity chromatography using an antibody against the protein itself.

Based on amino acid sequence information of the protein obtained above, the protein of one aspect of the present invention can be produced by a chemical synthesis method such as the Fmoc method (fluorenylmethyloxycarbonyl method) or the tBoc method (t-butyloxycarbonyl method). Alternatively, chemical synthesis can also be performed using a peptide synthesizer manufactured by Advanced ChemTech, Perkin-Elmer, Pharmacia, Protein Technology Instrument, Synthecell-Vega, Applied Biosystems, Shimadzu Corporation, or the like.

### 7. Method for Decomposing PET

Examples of a method for decomposing PET of one aspect of the present invention include a method using the protein of one aspect of the present invention. The method for decomposing PET according to one aspect of the present invention preferably includes, but is not limited to, decomposing PET in a reaction solution containing the protein according to one aspect of the present invention, the PET, magnesium chloride, and sodium carbonate.

A concentration of the protein of one aspect of the present invention in the reaction solution when decomposing the PET is preferably 1 µg/mL to 100 µg/mL, and more preferably 5 µg/mL to 20 µg/mL, from the viewpoint of PET decomposition efficiency, and the concentration is not limited thereto, and can be appropriately set according to an amount of PET to be decomposed and the like.

A concentration of the magnesium chloride in the reaction solution when decomposing the PET is preferably 0.05 mM to 10 mM, and more preferably 0.1 mM to 5 mM. The magnesium chloride contributes to stabilization of the protein of 1. above, and when the concentration of the magnesium chloride is 0.05 mM or more, the magnesium chloride has an advantage of enhancing the heat resistance of the protein, and when the concentration of the magnesium chloride is 10 mM or less, the magnesium chloride has an advantage that a binding ability of the protein to a substrate is hardly lowered.

A reaction temperature is preferably 40°C to 70°C, and more preferably 55°C to 65°C, from the viewpoint of the heat resistance or the PET decomposing activity of the protein of 1. above.

A pH during the reaction is preferably 6 to 11, and more preferably 8.5 to 9.5, from the viewpoint of the PET decomposing activity of the protein of 1. above.

A reaction time can be appropriately set depending on the amount of PET to be decomposed and the like, and is preferably 12 hours to 48 hours. When the treatment is performed for a long time, the protein of 1. above may be added periodically.

When the protein of one aspect of the present invention is used to decompose the PET, a form of the PET to be decomposed is not limited, and examples thereof include fibrous, granular, flake, pellet, film, lump, and bottle-shaped PET. A mixture thereof can also be used.

Waste such as PET bottles can be treated with the protein of one aspect of the present invention. The PET can be decomposed by the protein of one aspect of the present invention, and decomposition products can be recycled. Further, modification of a surface of a PET processed product such as a PET film, surface modification of PET fibers, washing of clothes using PET fibers, washing of a PET resin to be recycled, and the like can also be performed using the protein of one aspect of the present invention.

### 8. Method for Producing at Least One of TPA and MHET

Examples of a method for producing at least one of TPA and MHET of one aspect of the present invention include a method including decomposing PET using the protein of one aspect of the present invention. The method for producing at least one of TPA and MHET of one aspect of the present invention preferably includes, but is not limited to, decomposing PET in a reaction solution containing the protein of one aspect of the present invention, the PET, magnesium chloride, and sodium carbonate.

The protein of one aspect of the present invention can decompose a main chain of PET. Therefore, the protein of one aspect of the present invention can decompose BHET, which is an intermediate product of PET decomposition, into MHET, and can also decompose MHET into TPA and EG. That is, the protein of one aspect of the present invention can hydrolyze PET or BHET, which is a partial structure of PET, as a substrate to produce MHET, and further produce TPA and EG.

A concentration of the protein of one aspect of the present invention in the reaction solution, a concentration of the magnesium chloride in the reaction solution when decomposing the PET, a reaction temperature, a pH during the reaction, a reaction time, and a form of PET to be decomposed are as described in 7. above.

### [Analysis Example]

In Examples, TPA and MHET were analyzed and quantified by the following procedures.

A solution after an enzyme reaction was centrifuged, and a supernatant was collected. TPA and MHET contained in the supernatant were analyzed by HPLC (manufactured by Shimadzu Corporation).

### [Analysis Conditions]

Column: YMC-Triart C18/S-5 µm/12 nm, 150 mm × 4.6 mm (manufactured by YMC Co., Ltd.)
Column temperature: 25°C
Mobile phase: 0.1% formic acid, 20% acetonitrile (v/v)
Flow rate: 0.8 mL/min
Detection wavelength: 260 nm

### [Example]

Hereinafter, the present invention will be specifically described with reference to Examples, but the present invention is not limited to the following Examples as long as the gist thereof is not exceeded.

### [Example 1] Construction of Mutant-type PET2 Expressing Strain

### (1) Construction of Expression Plasmid of Wild-type PET2

PCR was performed using, as a template, a DNA (SEQ ID NO: 2) consisting of the nucleotide sequence of a gene encoding the amino acid sequence (SEQ ID NO: 1) of the wild-type PET2 described in Non Patent Literature 2 and using DNAs consisting of the nucleotide sequences represented by SEQ ID NOs: 5 and 6 as a primer set to obtain a DNA fragment of PET2. A DNA represented by SEQ ID NO: 2 was prepared by artificial synthesis. The DNA fragment of the wild-type PET2 obtained above and an expression vector pET28a (manufactured by Sigma-Aldrich) were ligated using an In-Fusion HD Cloning Kit (manufactured by Takara Bio Inc.) to obtain pET28a-PET2, which is an expression plasmid of the wild-type PET2.

### (2) Construction of Expression Plasmid of Mutant-type PET2 according to Present Embodiment - 1

PCR was performed using the plasmid pET28a-PET2 obtained in the above (1) as a template and DNAs consisting of the nucleotide sequences represented by SEQ ID NOs: 7 and 8 as a primer set to prepare pET28a-L103K, which is an expression plasmid of the mutant-type PET2 according to the present embodiment in which the L-leucine residue at position 103 was substituted with an L-lysine residue in the amino acid sequence of PET2 represented by SEQ ID NO: 1.

Next, PCR was performed using the pET28a-L103K obtained above as a template and DNAs consisting of the nucleotide sequences represented by SEQ ID NOs: 9 and 10 as a primer set to prepare pET28a-L103K/G76C , which is an expression plasmid of the mutant-type PET2 according to the present embodiment in which the L-leucine residue at position 103 was substituted with an L-lysine residue and the L-glycine residue at position 76 was substituted with an L-cysteine residue in the amino acid sequence of PET2.

Similarly, PCR was performed using the plasmid obtained by the previous operation as a template and DNAs consisting of nucleotide sequences corresponding to each SEQ ID NO illustrated in Table 1 as a primer set to sequentially produce expression plasmids of the mutant-type PET2 according to the present embodiment in which a mutation was added to the amino acid sequence of PET2. Table 1 illustrates each of added mutation sites and SEQ ID NOs of the corresponding primer sets.

### [Table 1]

**Table 1**

| Mutation site | SEQ ID NO of primer set |
|---|---|
| L103K (substitution of L-leucine residue at position 103 with L-lysine residue) | SEQ ID NO: 7 and SEQ ID NO: 8 |
| G76C (substitution of L-glycine residue at position 76 with L-cysteine residue) | SEQ ID NO: 9 and SEQ ID NO: 10 |
| A144C (substitution of L-alanine residue at position 144 with L-cysteine residue) | SEQ ID NO: 11 and SEQ ID NO: 12 |
| Q134G (substitution of L-glutamine residue at position 134 with L-glycine residue) | SEQ ID NO: 13 and SEQ ID NO: 14 |
| A222M (substitution of L-alanine residue at position 222 with L-methionine residue) | SEQ ID NO: 15 and SEQ ID NO: 16 |

### (3) Construction of Expression Plasmid of Mutant-type PET2 According to Present Embodiment - 2

Using, as a template, an expression plasmid (pET28a-L103K/G76C/A144C/Q134G/A222M) of the mutant-type PET2 according to the present embodiment to which all mutations illustrated in Table 1 were added, and DNAs consisting of the nucleotide sequences represented by SEQ ID NOs: 5 and 6 as a primer set, error-prone PCR was performed using a Diversify PCR Random Mutagenesis Kit (manufactured by Takara Bio Inc.). As a result, a DNA fragment of the mutant-type PET2 according to the present embodiment to which a new mutation site L73Q (substitution of the L-leucine residue at position 73 with an L-glutamine residue) was added was obtained.

The obtained DNA fragment and the expression vector pET28a were ligated using an In-Fusion HD Cloning Kit to prepare an expression plasmid of the mutant-type PET2 according to the present embodiment in which mutations of L103K, G76C, A144C, Q134G, A222M, and L73Q were added to the amino acid sequence of PET2.

Next, error-prone PCR was performed in the same manner as described above using the above-obtained plasmid (pET28a-L103K/G76C/A144C/Q134G/A222M/L73Q) as a template and DNAs consisting of the nucleotide sequences represented by SEQ ID NOs: 5 and 6 as a primer set to obtain a DNA fragment of the mutant-type PET2 according to the present embodiment to which a mutation site I203T (substitution of the L-isoleucine residue at position 203 with an L-threonine residue) was newly added. The obtained DNA fragment and the expression vector pET28a were ligated to prepare an expression plasmid of the mutant-type PET2 according to the present embodiment in which mutations of L103K, G76C, A144C, Q134G, A222M, L73Q, and I203T were added to the amino acid sequence of PET2.

### (4) Construction of Expression Plasmid of Comparative Mutant-type PET2

PCR was performed using the plasmid pET28a-PET2 obtained in the above (1) as a template and DNAs consisting of the nucleotide sequences represented by SEQ ID NOs: 17 and 18 as a primer set to prepare pET28a-W254F, which is an expression plasmid of the comparative mutant-type PET2 in which the L-tryptophan residue at position 254 was substituted with an L-phenylalanine residue in the amino acid sequence of PET2 represented by SEQ ID NO: 1.

PCR was performed using the plasmid pET28a-PET2 obtained in the above (1) as a template and DNAs consisting of the nucleotide sequences represented by SEQ ID NOs: 19 and 20 as a primer set to prepare pET28a-N257D, which is an expression plasmid of the comparative mutant-type PET2 in which the L-asparagine residue at position 257 was substituted with an L-aspartic acid residue in the amino acid sequence of PET2 represented by SEQ ID NO: 1.

PCR was performed using the plasmid pET28a-PET2 obtained in the above (1) as a template and DNAs consisting of the nucleotide sequences represented by SEQ ID NOs: 21 and 22 as a primer set to prepare pET28a-L103D, which is an expression plasmid of the comparative mutant-type PET2 in which the L-leucine residue at position 103 was substituted with an L-aspartic acid residue in the amino acid sequence of PET2 represented by SEQ ID NO: 1.

### [Table 2]

**Table 2**

| Mutation site | SEQ ID NO of primer set |
|---|---|
| W254F (substitution of L-tryptophan residue at position 254 with L-phenylalanine residue) | SEQ ID NO: 17 and SEQ ID NO: 18 |
| N257D (substitution of L-asparagine residue at position 257 with L-aspartic acid residue) | SEQ ID NO: 19 and SEQ ID NO: 20 |
| L103D (substitution of L-leucine residue at position 103 with L-aspartic acid residue) | SEQ ID NO: 21 and SEQ ID NO: 22 |

### (5) Construction of Transformants Carrying Various PET2 Expression Plasmids

Each of the plasmids constructed in (1) to (4) was transformed into SHuffle T7 Express Competent E. coli (New England BioLabs) to obtain a total of 12 transformants (PET2 strain, L103K strain, L103K/G76C strain, L103K/A144C strain, L103K/G76C/A144C strain, L103K/G76C/A144C/Q134G strain, L103K/G76C/A144C/Q134G/A222M strain, L103K/G76C/A144C/Q134G/A222M/L73Q strain, L103K/G76C/A144C/Q134G/A222M/L73Q/I203T strain, W254F strain, N257D strain, and L103D strain).

### [Example 2] Evaluation of Each Mutation Site Based on Measurement of PET Decomposing Activity of Various PET2 Purified Enzymes

### (1) Acquisition of Various PET2 Purified Enzymes

The total of 12 transformants obtained in Example 1 (5) were cultured on an LB plate at 30°C for 24 hours, inoculated into a large test tube containing 5 mL of LB medium containing 100 mg/L kanamycin, and shake-cultured at 30°C for 20 hours. Thereafter, 0.6 mL of the obtained culture solution was transferred to a flask containing 30 mL of LB medium containing 100 mg/L of kanamycin, and shake-cultured at 37°C. After a bacteria cell OD600 reached a range of 0.4 to 0.6, isopropyl-β-D-thiogalactopyranoside (IPTG) was added to a final concentration of 0.5 mM, followed by shaking culture for 20 hours. After completion of the culture, the culture solution was centrifuged to remove the supernatant, and bacteria cells were collected.

A crude protein extract was obtained from the obtained bacteria cells by a standard method, and various PET2 enzyme solutions were collected from the extract using TALON Metal Affinity Resin (manufactured by Takara Bio Inc.). The enzyme solution was diluted with 100 mM sodium carbonate buffer (pH 9.2), and concentrated and desalted using Amicon Ultra-4 (manufactured by Merck Millipore) to obtain various PET2 purified enzymes.

### (2) Measurement of PET Decomposing Activity

The PET decomposing activity of various PET2 purified enzymes was measured by the following enzyme reaction, and the effects of introduction of each mutation site were compared.

A 0.5 mL reaction solution containing 5 µg of each PET2 purified enzyme, 100 mM sodium carbonate buffer (pH 9.2), 1 mM or 10 mM magnesium chloride, and 0.01 g of PET film fragment (A-PET manufactured by Mineron Chemical Industry Co., Ltd.) was prepared, and the enzyme reaction was performed at 60°C for 24 hours. After completion of the reaction, TPA and MHET produced by PET decomposition were analyzed by HPLC. Table 3 illustrates mutation sites of various PET2 purified enzymes, a concentration (mM) of magnesium chloride (MgCl₂) in the reaction solution, concentrations (mM) of TPA and MHET produced by the enzyme reaction of various PET2 purified enzymes, and a total value (mM) thereof.

### [Table 3]

**Table 3**

| | Mutation site | MgCl₂ [mM] | TPA [mM] | MHET [mM] | Total [mM] |
|---|---|---|---|---|---|
| Wild type | - | 10.0 | 0.2 | 0.0 | 0.2 |
| Mutant type according to present embodiment | L103K | 10.0 | 2.0 | 0.3 | 2.3 |
| Mutant type according to present embodiment | L103K/G76C | 10.0 | 1.1 | 0.2 | 1.3 |
| Mutant type according to present embodiment | L103K/A144C | 10.0 | 0.5 | 0.1 | 0.6 |
| Mutant type according to present embodiment | L103K/G76C/A144C | 10.0 | 3.9 | 0.9 | 4.8 |
| Mutant type according to present embodiment | L103K/G76C/A144C/Q134G | 10.0 | 8.0 | 2.5 | 10.5 |
| Mutant type according to present embodiment | L103K/G76C/A144C/Q134G /A222M | 10.0 | 8.7 | 3.7 | 12.4 |
| Mutant type according to present embodiment | L103K/G76C/A144C/Q134G /A222M/L73Q | 10.0 | 11.1 | 8.0 | 19.1 |
| Mutant type according to present embodiment | L103K/G76C/A144C/Q134G /A222M/L73Q/I203T | 10.0 | 11.7 | 8.2 | 19.9 |
| Mutant type according to present embodiment | L103K/G76C/A144C/Q134G /A222M/L73Q/I203T | 1.0 | 16.6 | 17.1 | 33.7 |
| Comparative mutant type | W254F | 10.0 | 0.1 | 0.0 | 0.1 |
| Comparative mutant type | N257D | 10.0 | 0.1 | 0.0 | 0.1 |
| Comparative mutant type | L103D | 10.0 | 0 | 0 | 0 |

As illustrated in Table 3, the concentrations of TPA and MHET produced by the enzyme reaction, and the total value thereof (hereinafter, referred to as a decomposition amount) were increased in the purified enzymes of the mutant-type PET2 according to any of the embodiments of the present invention, compared to the purified enzyme of the wild-type PET2 and the purified enzyme of the comparative mutant-type PET2, indicating that the introduction of the mutation site improved the PET decomposing activity. In particular, for the purified enzyme into which the mutation site L73Q was introduced, the decomposition amount was increased by 6.7 mM as compared with the purified enzyme into which the mutation was not introduced, and it was shown that the mutation site greatly contributed to the improvement of the PET decomposing activity. Similarly, for the purified enzyme into which the mutation site Q134G was introduced, the decomposition amount was increased by 5.7 mM as compared with the purified enzyme into which the mutation was not introduced, and it was shown that the mutation site greatly contributed to the improvement of the PET decomposing activity. As for the mutation site A144C, the decomposition amount increased by 2.5 mM by introducing the mutation site A144C in combination with the mutation site G76C, and it was shown that the combination of the mutation sites A144C and G76C greatly contributed to the improvement of the PET decomposing activity.

Further, the decomposition amount was greater when the concentration of the magnesium chloride in the reaction solution was 1 mM than when the concentration of the magnesium chloride was 10 mM, indicating that this is a suitable concentration for the decomposition of PET by the mutant-type PET2 according to the present embodiment.

### [Example 3] Comparison of PET Decomposing Activity Between Previously Reported Mutant-type PET2 and Mutant-type PET2 According to Present Embodiment

### (1) Construction of Expression Plasmid of Previously Reported Mutant-type PET2

As a previously reported mutant-type PET2, PCR was performed using, as a template, a DNA (SEQ ID NO: 4) consisting of the nucleotide sequence of a gene encoding a mutant-type PET2 (SEQ ID NO: 3) (hereinafter, referred to as PET2s) described in Non Patent Literature 3 and DNAs consisting of the nucleotide sequences represented by SEQ ID NOs: 23 and 24 as a primer set to obtain a DNA fragment of PET2s. A DNA represented by SEQ ID NO: 4 was prepared by artificial synthesis.

The DNA fragment of PET2s obtained above and an expression vector pET28a were ligated using an In-Fusion HD Cloning Kit to obtain pET28a-PET2s, which is an expression plasmid of PET2s.

### (2) Construction of Microorganism Carrying Expression Plasmid of PET2s

The plasmid constructed in (1) was transformed into SHuffle T7 Express Competent E. coli to obtain a PET2s strain.

### (3) Acquisition of Purified Enzyme of PET2s

The transformant obtained in (2) was cultured on an LB plate at 30°C for 24 hours, inoculated into a large test tube containing 5 mL of LB medium containing 100 mg/L kanamycin, and shake-cultured at 30°C for 20 hours. Thereafter, 0.6 mL of the obtained culture solution was inoculated into a flask containing 30 mL of LB medium containing 100 mg/L kanamycin, and shake-cultured at 37°C. After a bacterial cell OD600 reached a range of 0.4 to 0.6, IPTG was added to a final concentration of 0.5 mM, followed by shaking culture for 20 hours. After completion of the culture, the culture solution was centrifuged to remove a supernatant, and bacterial cells were collected.

A crude protein extract was obtained from the obtained bacterial cells by a standard method, and an enzyme solution of PET2s was collected from the extract using TALON (registered trademark) Metal Affinity Resin (manufactured by Takara Bio Inc.). The enzyme solution was diluted with 100 mM sodium carbonate buffer (pH 9.2), and concentrated and desalted using Amicon (registered trademark) Ultra- (manufactured by Merck Millipore) to obtain a purified enzyme of PET2s.

### (4) Evaluation of PET Decomposing Activity

The PET decomposing activity of the purified enzyme of PET2s and the purified enzymes of the four types of mutant-type PET2 according to the present embodiment obtained in Example 1 were measured by the following enzyme reaction.

A 0.5 mL of a reaction solution containing 5 µg of each purified enzyme, 100 mM sodium carbonate buffer (pH 9.2), 10 mM magnesium chloride, and 0.01g of PET film fragments was prepared, and an enzyme reaction was performed at 60°C for 24 hours. After completion of the reaction, TPA and MHET produced by PET decomposition were analyzed by HPLC. Table 4 illustrates mutation sites of various PET2 purified enzymes, concentrations (mM) of TPA and MHET produced by the enzyme reaction of each purified enzyme, and a total value (mM) thereof (a decomposition amount).

### [Table 4]

**Table 4**

| Mutation site/enzyme name | TPA [mM] | MHET [mM] | Total [mM] |
|---|---|---|---|
| L103K/G76C/A144C/Q134G | 8.0 | 2.5 | 10.5 |
| L103K/G76C/A144C/Q134G/A222M | 8.7 | 3.7 | 12.4 |
| L103K/G76C/A144C/Q134G/A222M/L73Q | 11.1 | 8.0 | 19.1 |
| L103K/G76C/A144C/Q134G/A222M/L73Q/I203T | 11.7 | 8.2 | 19.9 |
| PET2s | 7.4 | 1.5 | 8.9 |

As illustrated in Table 4, the purified enzyme of the mutant-type PET2 according to the present embodiment into which the mutations L103K, G76C, A144C, and Q134G were introduced, the purified enzyme of the mutant-type PET2 according to the present embodiment into which the mutations L103K, G76C, A144C, Q134G, and A222M are introduced, the purified enzyme of the mutant-type PET2 according to the present embodiment into which the mutations L103K, G76C, A144C, Q134G, A222M, and L73Q are introduced, and the purified enzyme of the mutant-type PET2 according to the embodiment into which the mutations L103K, G76C, A144C, Q134G, A222M, L73Q, and I203T are introduced all showed a decomposition amount and PET decomposing activity higher than those of the PET2s.

Although various embodiments have been described above, the present invention is not limited to these examples. It is apparent to those skilled in the art that various modifications or alterations can be conceived within the scope described in the claims, and it is understood that the modifications or alterations naturally fall within the technical scope of the present invention. The components in the above-described embodiments may be combined in any manner without departing from the spirit of the invention.

The present application is based on a Japanese patent application filed on December 9, 2022 (patent application No. 2022-197409), which is hereby incorporated by reference in its entirety.

### INDUSTRIAL APPLICABILITY

Since the protein according to one aspect of the present invention includes amino acid residue substitution at a specific site, the protein has a PET decomposing activity higher than that of a protein having no amino acid residue substitution.

### SEQUENCE LISTING FREE TEXT

SEQ ID NO: 1: amino acid sequence of PET2
SEQ ID NO: 2: nucleotide sequence of PET2
SEQ ID NO: 3: amino acid sequence of PET2s
SEQ ID NO: 4: nucleotide sequence of PET2s
SEQ ID NO: 5: nucleotide sequence of primer Fw for amplifying PET2 fragment
SEQ ID NO: 6: nucleotide sequence of primer Rv for amplifying PET2 fragment
SEQ ID NO: 7: nucleotide sequence of primer Fw for amplifying L103K fragment
SEQ ID NO: 8: nucleotide sequence of primer Rv for amplifying L103K fragment
SEQ ID NO: 9: nucleotide sequence of primer Fw for amplifying G76C fragment
SEQ ID NO: 10: nucleotide sequence of primer Rv for amplifying G76C fragment
SEQ ID NO: 11: nucleotide sequence of primer Fw for amplifying A144C fragment
SEQ ID NO: 12: nucleotide sequence of primer Rv for amplifying A144C fragment
SEQ ID NO: 13: nucleotide sequence of primer Fw for amplifying Q134G fragment
SEQ ID NO: 14: nucleotide sequence of primer Rv for amplifying Q134G fragment
SEQ ID NO: 15: nucleotide sequence of primer Fw for amplifying A222M fragment
SEQ ID NO: 16: nucleotide sequence of primer Rv for amplifying A222M fragment
SEQ ID NO: 17: nucleotide sequence of primer Fw for amplifying W254F fragment
SEQ ID NO: 18: nucleotide sequence of primer Rv for amplifying W254F fragment
SEQ ID NO: 19: nucleotide sequence of primer Fw for amplifying N257D fragment
SEQ ID NO: 20: nucleotide sequence of primer Rv for amplifying N257D fragment
SEQ ID NO: 21: nucleotide sequence of primer Fw for amplifying L103D fragment
SEQ ID NO: 22: nucleotide sequence of primer Rv for amplifying L103D fragment
SEQ ID NO: 23: nucleotide sequence of primer Fw for amplifying PET2s fragment
SEQ ID NO: 24: nucleotide sequence of primer Rv for amplifying PET2s fragment

## Claims

1. A protein consisting of an amino acid sequence in which at least one modification selected from the group consisting of the following [1] to [7] is introduced in the amino acid sequence represented by SEQ ID NO: 1:
[1] a modification in which the amino acid residue at a position 103 is substituted with a lysine residue,
[2] a modification in which the amino acid residue at a position 76 is substituted with a cysteine residue,
[3] a modification in which the amino acid residue at a position 144 is substituted with a cysteine residue,
[4] a modification in which the amino acid residue at a position 134 is substituted with a glycine residue,
[5] a modification in which the amino acid residue at a position 222 is substituted with a methionine residue,
[6] a modification in which the amino acid residue at a position 73 is substituted with a glutamine residue, and
[7] a modification in which the amino acid residue at a position 203 is substituted with a threonine residue.

2. A protein consisting of an amino acid sequence in which at least one modification selected from the group consisting of the following [1'] to [7'] is introduced in an amino acid sequence of a mutant protein (B) having a modification which is at least one of deletion, substitution, insertion, and addition of 1 to 20 amino acid residues with respect to the amino acid sequence represented by SEQ ID NO: 1 and having a polyethylene terephthalate (PET) decomposing activity higher than that of the mutant protein (B):
[1'] a modification in which an amino acid residue corresponding to the amino acid residue at a position 103 in SEQ ID NO: 1 is substituted with a lysine residue,
[2'] a modification in which an amino acid residue corresponding to the amino acid residue at a position 76 in SEQ ID NO: 1 is substituted with a cysteine residue,
[3'] a modification in which an amino acid residue corresponding to the amino acid residue at a position 144 in SEQ ID NO: 1 is substituted with a cysteine residue,
[4'] a modification in which an amino acid residue corresponding to the amino acid residue at a position 134 in SEQ ID NO: 1 is substituted with a glycine residue,
[5'] a modification in which an amino acid residue corresponding to the amino acid residue at a position 222 in SEQ ID NO: 1 is substituted with a methionine residue,
[6'] a modification in which an amino acid residue corresponding to the amino acid residue at a position 73 in SEQ ID NO: 1 is substituted with a glutamine residue, and
[7'] a modification in which an amino acid residue corresponding to the amino acid residue at a position 203 in SEQ ID NO: 1 is substituted with a threonine residue.

3. A protein consisting of an amino acid sequence in which at least one modification selected from the group consisting of the following [1"] to [7"] is introduced in an amino acid sequence of a homologous protein (C) having 80% or more identity with the amino acid sequence represented by SEQ ID NO: 1 and having a polyethylene terephthalate (PET) decomposing activity higher than that of the homologous protein (C):
[1"] a modification in which an amino acid residue corresponding to the amino acid residue at a position 103 in SEQ ID NO: 1 is substituted with a lysine residue,
[2"] a modification in which an amino acid residue corresponding to the amino acid residue at a position 76 in SEQ ID NO: 1 is substituted with a cysteine residue,
[3"] a modification in which an amino acid residue corresponding to the amino acid residue at a position 144 in SEQ ID NO: 1 is substituted with a cysteine residue,
[4"] a modification in which an amino acid residue corresponding to the amino acid residue at a position 134 in SEQ ID NO: 1 is substituted with a glycine residue,
[5"] a modification in which an amino acid residue corresponding to the amino acid residue at a position 222 in SEQ ID NO: 1 is substituted with a methionine residue,
[6"] a modification in which an amino acid residue corresponding to the amino acid residue at a position 73 in SEQ ID NO: 1 is substituted with a glutamine residue, and
[7"] a modification in which an amino acid residue corresponding to the amino acid residue at a position 203 in SEQ ID NO: 1 is substituted with a threonine residue.

4. A DNA encoding the protein according to any one of claims 1 to 3.

5. A recombinant DNA comprising the DNA according to claim 4.

6. A transformant obtained by transforming a host cell with the recombinant DNA according to claim 5.

7. A method for decomposing PET using the protein according to any one of claims 1 to 3.

8. The method for decomposing PET according to claim 7, comprising:
decomposing the PET in a reaction solution containing the protein according to any one of claims 1 to 3, the PET, magnesium chloride, and sodium carbonate.

9. The method for decomposing PET according to claim 8, wherein
a concentration of the magnesium chloride in the reaction solution is 0.1 mM to 5 mM.

10. A method for producing at least one of terephthalic acid (TPA) and monohydroxyethyl terephthalate (MHET), the method comprising:
decomposing PET using the protein according to any one of claims 1 to 3.

11. The method for producing at least one of TPA and MHET according to claim 10, comprising:
decomposing the PET in a reaction solution containing the protein according to any one of claims 1 to 3, the PET, magnesium chloride, and sodium carbonate.

12. The method for producing at least one of TPA and MHET according to claim 11, wherein
a concentration of the magnesium chloride in the reaction solution is 0.1 mM to 5 mM.
